# EUROPEAN PATENT SPECIFICATION

(11) **EP 2 716 290 B1**
(45) Date of publication and mention of the grant of the patent: **14.03.2018**
(21) Application number: 12789023.4
(22) Date of filing: 24.05.2012
(51) Int. Cl.: A61K 31/4412, A61K 9/28, A61K 31/513, A61K 31/53, A61K 47/26, A61K 47/32, A61K 47/36, A61K 47/38, A61P 35/00

(54) **DRY-COATED TABLET CONTAINING TEGAFUR, GIMERACIL AND OTERACIL POTASSIUM**
TROCKENBESCHICHTETE TABLETTE MIT TEGAFUR, GIMERACIL UND OTERACIL-KALIUM
COMPRIMÉ ENROBÉ À SEC CONTENANT DU TÉGAFUR, DU GIMÉRACIL ET DE L'OTÉRACIL POTASSIQUE

(30) Priority: 25.05.2011 JP 2011116884
(43) Date of publication of application: 09.04.2014
(73) Proprietor: Taiho Pharmaceutical Co., Ltd., Chiyoda-ku, Tokyo 101-8444 (JP)
(72) Inventor: OKAMOTO, Takumi, Tokushima-shi Tokushima 771-0194 (JP); YOSHIZAWA, Takashi, Tokushima-shi Tokushima 771-0194 (JP); OHNISHI, Yoshito, Tokushima-shi Tokushima 771-0194 (JP)
(74) Representative: Hoffmann Eitle
(86) International application number: PCT/JP2012/063260
(87) International publication number: WO 2012/161240

(56) References cited:
- EP-A1- 1 604 991
- EP-A1- 1 864 683
- EP-A1- 2 223 683
- WO-A1-92/21345
- WO-A1-03/028706
- CN-A- 1 660 105
- CN-A- 101 380 327
- CN-A- 101 711 765
- JP-A- 2003 533 470
- KAZUMASA IKEDA ET AL.: 'Ko Shuyozai 'TS-1' no Yakuri Sayo to Tainai Dotai' ANTIBIOTICS & CHEMOTHERAPY vol. 17, no. 7, 2001, pages 1318 - 1331, XP002904665
- HIROSHI FUKAYA ET AL.: 'Yugai Sayo Hatsugen to Funsai' CHOZAI TO JOHO vol. 14, no. 6, June 2008, pages 711 - 714, XP008171775

## Description

### Technical Field

The present invention relates to a dry-coated tablet for oral administration, comprising:an inner core containing, as active ingredients, (a) tegafur, (b) gimeracil, and (c) oteracil potassium, and an outer shell containing crystalline cellulose, wherein the dry-coated tablet is an orally disintegrating tablet.

### Background Art

A combination drug containing tegafur, gimeracil, and oteracil potassium is an antitumor agent that has a feature of low toxicity against digestive organs while having increased antitumor effect, and is obtained by blending tegafur, which is a prodrug of fluorouracil (5-FU), with gimeracil, which is a degradation inhibitor for 5-FU, and oteracil potassium, which is a phosphorylation inhibitor. The agent is widely used in the clinical field as an orally administrable cancer chemotherapy agent (Patent Literature 1).

At present, a combination drug containing tegafur, gimeracil, and oteracil potassium is commercially available in granular form under the name of "TS-1 combination granule"; and as a capsule, under the name of "TS-1 combination capsule," containing tegafur:gimeracil:oteracil potassium at a molar ratio of 1:0.4:1 (Patent Literature 2). However, since capsules and granules are sometimes difficult to swallow for elderly people, who have reduced swallowing function, there has been a demand in the clinical field for an orally disintegrating tablet that immediately disintegrates in the mouth and that can be easily ingested, even by a person who has difficulty swallowing.

Generally, active ingredients showing antitumor effect often have higher pharmacological activity, as they are classified as powerful drugs. Tegafur is one of these, and a great deal of caution is required in the handling thereof. In the event that an active ingredient of an anticancer agent is uncovered, or an active ingredient is scattered due to the preparation being cracked or chipped, the possibility of drug exposure may extend not only to medical workers, but also to patients who are taking the medicine and people providing assistance in taking the medicine. Generally, techniques used in order to lessen drug exposure include encapsulating a medical agent in a capsule consisting of gelatin or the like; and coating a medical agent with sugar, polymer, or the like, to form a tablet. However, since these techniques cause a delay in the disintegration of the pharmaceutical preparation, immediate disintegration in the mouth cannot be expected.

In addition, an attempt to improve the disintegration ability of an orally disintegrating tablet often leads to a pharmaceutical preparation having low strength. Such a preparation cannot withstand manufacturing and distribution processes, as well as dispensation using an automatic dividing and packing machine in which a medical agent is divided into portions by being dropped from a height of ordinarily about 1 m. This results in the preparation becoming cracked or chipped, and concerns regarding an increase of medical agent exposure risk to medical care workers, etc.

As a pharmaceutical preparation containing tegafur, gimeracil, and oteracil potassium, for example, Patent Literature 3 discloses a combination drug containing tegafur, gimeracil, and oteracil potassium that is orally administrable and stable under humidified conditions. However, the formulated preparation is intended to have improved stability as a preparation, and does not take into consideration ingestibility, etc., for patients who have difficulty swallowing. Furthermore, addition of a disintegrant and the like to a sugar, which is considered stable, for improving tablet functions such as disintegration, results in destabilization of the active ingredient. Furthermore, Patent Literature 4 discloses an orally disintegrating combination tablet containing tegafur, gimeracil, and oteracil potassium. However, this patent does not take any measures against drug exposure to medical care workers, caregivers, etc., even though the present drug is an anticancer agent with high activity.

### Citation List

### Patent Literature

PTL 1: JP2614164B2
PTL 2: WO2009084216A1 discloses a tablet comprising (a) tegafur, (b) gimeracil, and (c) oteracil potassium, in the core and comprises a "coating with a saccharide other than a cellulose derivative"(§008). The cellulose derivative so called disintegrant is in the core : HPC.
   PTL 2 mentions an Orally disintegration tablet as an option.
PTL 3: JP2010235539A
PTL 4: CN1660105A
PTL 5: EP1604991A1 discloses an antitumor effect potentiator containing a therapeutically effective amount of Tegafur, Gimeracil in an amount effective for enhancing the antitumor effect and Oteracil potassium in an amount effective in inhibiting a side effect, characterized by containing at least one component selected from the group consisting of folinic acid and its pharmaceutically acceptable salts in an amount effective for enhancing the antitumor effect as the active ingredient.
PTL 6: EP1864683A1 discloses a radiotherapy enhancer comprising (A) tegafur and (B) gimeracil that can reduce the radiation dose and adverse drug reactions when used in combination with cancer radiotherapy.

### Summary of Invention

### Technical Problem

As described above, in response to hitherto known combination drugs containing tegafur, gimeracil, and oteracil potassium, there is a desire for a drug that sufficiently reduces drug exposure risk, has sufficient tablet strength, and does not result in decrease in medication adherence when taken.

An object of the present invention is to provide a dry-coated combination orally disintegrating tablet containing tegafur, gimeracil, and oteracil potassium. The tablet achieves reduction in drug exposure risk and immediate disintegration, and has sufficient tablet strength. Specifically, an object of the present invention is to provide a dry-coated tablet that has sufficient mechanical strength, achieves reduction in drug exposure risk, and immediately disintegrates in the mouth; this is enabled by combining an outer shell having fine compactibility and excellent disintegration ability, and an inner core containing an active ingredient.

### Solution to Problem

In order to solve the above-described problem, the present inventors made attempts to manufacture various orally administrable combination tablets containing tegafur, gimeracil, and oteracil potassium.

As a result, they developed a preparation that has sufficient mechanical strength, achieves reduction in drug exposure risk, and immediately disintegrates in the mouth, by forming a dry-coated tablet by combining an inner core containing tegafur, gimeracil, and oteracil potassium; and an outer shell containing cristalline cellulose, and having fine compactability and excellent disintegration ability. The present inventors thereby perfected the present invention.

Thus, the present invention provides a dry-coated tablet as follows.
(1) A dry-coated tablet comprising: an inner core containing, as active ingredients, (a) tegafur, (b) gimeracil, and (c) oteracil potassium; and an outer shell containing crystalline cellulose, wherein the dry-coated tablet is an orally disintegrating tablet.
(2) The dry-coated tablet according to (1), containing (a) tegafur, (b) gimeracil, and (c) oteracil potassium at a molar ratio of 1:0.4:1.
(3) The dry-coated tablet according to (1) or (2), wherein the active ingredients consisting of (a) tegafur, (b) gimeracil, and (c) oteracil potassium account for 50 mass% to 100 mass% of components of the inner core, preferably 70 mass% to 99 mass%.
(4) The dry-coated tablet according to any one of (1) to (43), wherein the total content of the active ingredients (a) to (c) accounts for 10 to 60 mass% of the dry-coated tablet.
(5) The dry-coated tablet according to any one of (1) to (54), wherein the outer shell contains one or two compounds among lactose and hydroxypropyl cellulose.
(6) The dry-coated tablet according to (5), wherein the outer shell contains lactose.
(7) The dry-coated tablet according to (5) or (6) wherein the outer shell further contains a disintegrant.
(8) The dry-coated tablet according to (7), wherein the disintegrant is selected from the group consisting of crospovidone, carmellose, corn starch, low-substituted hydroxypropyl cellulose, and partially pregelatinized starch, preferably crospovidone and/or partially pregelatinized starch.
(9) The dry-coated tablet according to any one of (1) to (8), wherein the outer shell contains 30 to 65 mass% lactose.
(10) The dry-coated tablet according to any one of (1) to (9), wherein the outer shell contains 30 to 50 mass% crystalline cellulose.
(11) The dry-coated tablet according to any one of (1) to (10), wherein the outer shell contains crospovidone by 2.5 to 15 mass%.
(12) The dry-coated tablet according to any one of (1) to (11), wherein the outer shell contains 2 to 7.5 mass% partially pregelatinized starch.

### Advantageous Effects of Invention

With the present invention, since a drug is confined in the inner core to form the dry-coated tablet and the active ingredients do not exist on the surface of the tablet, it becomes possible to greatly reduce the risk of drug exposure for medical care workers, etc., and achieve adequate tablet strength and immediate disintegration. Furthermore, since it is possible to reduce contact between the active ingredients and an additive that promotes degradation thereof by separating the inner core and the outer shell, degradation of the active ingredients can be suppressed.

### Brief Description of Drawings

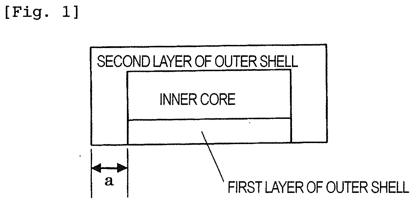

Fig. 1 is a schematic diagram of a cross section of a dry-coated tablet, as claimed.

### Description of Embodiments

In the present invention, although the molar ratio of tegafur, gimeracil, and oteracil potassium can be appropriately selected, a combination drug containing those ingredients at a molar ratio of 1:0.4:1 is preferable. The individual amounts of tegafur, gimeracil, and oteracil potassium, which are active ingredients in the dry-coated tablet of the present invention, change depending on the pharmaceutical form, administration schedule, etc. The amounts are not particularly limited, and can be appropriately selected. However, the total content of the three active ingredients in the dry-coated tablet is preferably about 10 to 60 mass%, more preferably 20 to 50 mass%, and particularly preferably 25 to 35 mass%.

A preferable dry-coated tablet of the present invention has an outer shell having a friability (accumulative number of rotations: 100 rotations) of not higher than 0.3%; and a more preferable dry-coated tablet has an outer shell having a friability of not higher than 0.1%. Here, "friability" refers to the same term ordinarily used in the technical field of pharmaceutical preparations. That is, "friability" is an index of tablet strength, and a parameter for evaluating the amount of reduction of tablet weight with a friability testing machine using a rotating drum in order to understand whether a molded tablet can withstand vibration and impact during subsequent steps of coating, printing, packaging, and market distribution. More specifically, friability is obtained in accordance with the "Tablet Friability Test" described in the reference information of the revised fifteenth edition of the Japanese Pharmacopoeia, by adjusting the number of rotations of a drum (internal diameter 287±4 mm) with an electric motor to, for example, 24 to 26 rotations per 1 minute; measuring the tablet weight after a certain number of rotations; and calculating the amount of reduction in percentage with respect to the starting tablet weight. In the present specification, friability obtained when the accumulative number of rotations is set at 100 rotations is used, and is calculated from a tablet weight obtained after 4 minutes at 25 rpm. The tablet strength is defined as being high when the friability obtained after 100 rotations in accumulative number of rotations is not higher than 0.3%. Comparatively, the tablet strength is defined as being low when the friability is higher than 0.3%. Since the active ingredients contained in the present invention act as an anticancer agent, and since it is necessary to increase the tablet strength in order to suppress risk of exposure to be lower than a general medical agent, friability is preferably set to be lower than that of an ordinarily preparation.

The dry-coated tablet of the present invention is preferably a dry-coated tablet having an outer shell that substantially does not become cracked or chipped after a drop test from a height of 1 m, and more preferably is a dry-coated tablet having an outer shell that substantially does not become cracked or chipped after a drop test from a height of 2 m. Here, "drop test" is a test ordinarily used in the technical field of pharmaceutical preparations in order to understand whether a preparation can withstand vibration and impact during manufacturing, distribution, etc.; or upon dispensation by an automatic dividing and packing machine. More specifically, a tablet is caused to undergo a gravity-drop onto a stainless steel plate from a height of 1 m to 2 m to examine whether the tablet becomes cracked or chipped, and measure the reduction rate of mass of the tablet before and after the test. Furthermore, in the present invention, "substantially does not become cracked or chipped" refers to, after a drop test is performed on five tablets, not being cracked or chipped at all and having a mass reduction rate of lower than 0.1% for the five tablets.

The dry-coated tablet of the present invention preferably has a disintegration time of not more than 120 seconds for the entire dry-coated tablet. More specifically, it is a dry-coated tablet having a disintegration time of not more than 120 seconds, which is obtained by conducting a disintegration test in accordance with the Disintegration Test described in the General Tests, Processes and Apparatus section in the revised fifteenth edition of the Japanese Pharmacopoeia. The disintegration time is preferably not more than 95 seconds, and further preferably not more than 80 seconds. When a dry-coated tablet with rapid oral disintegration characteristics is manufactured, the actual dissolution/disintegration time in the mouth is equal to or sometimes faster than the disintegration time obtained by the disintegration test due to friction in the mouth and additional motion by the tongue. Next, the disintegration test is briefly summarized. A tester is attached on a receive shaft, placed in a beaker, and adjusted so as to be able to smoothly perform an up-and-down motion for 29 to 32 round trips in one minute at an amplitude of 53 to 57 mm. The tester is set such that, at the lowest position for the tester, a mesh surface at the bottom is located 25 mm from the bottom of the beaker; and the amount of water added to the beaker is set such that, at the lowest position for the tester, the top surface of the tester matches the upper surface of the liquid. The temperature of the liquid is maintained at 37±2°C. A single sample tablet is placed in a glass tube of the tester, an auxiliary disk is added thereto, and, using water as a test liquid, an up-and-down motion is performed. The time required until residues of the sample are not observed within the glass tube is measured as the disintegration time.

Disintegration of a tablet in the mouth greatly depends on a quantity of water as small as that existing in saliva, and the slight grinding power between the tongue and the upper jaw. Thus, the disintegration test (the Disintegration Test described in the General Tests, Processes and Apparatus section in the revised fourteenth edition of the Japanese Pharmacopoeia) such as those commonly used on tablets does not reflect the actual disintegration in the mouth. Therefore, ODT-101 (manufactured by Toyama Sangyo Co., Ltd.) and Tricorptester (manufactured by Okada Seiko Co., Ltd.) were developed and sold as orally disintegrating tablet testers; using these, it is possible to easily perform measurements with high reproducibility without individual differences, considering the correlation with data from organoleptic evaluation test performed on human (cf. Narazaki R., Harada T., et al., Chem. Pharm. Bull., 52, 704-707 (2004)). The disintegration time of the dry-coated tablet of the present invention measured by the orally disintegrating tablet tester ODT-101 is preferably not more than 20 seconds, more preferably not more than 16 seconds, and particularly preferably not more than 14 seconds.

The dry-coated tablet of the present invention preferably has a dissolution rate (CDHP, 15-minute value) measured by Method 2 (50 min⁻¹, test liquid: 900 mL of water) of the Dissolution Test described in the General Tests, Processes and Apparatus section of the Japanese Pharmacopoeia of not lower than 90%, more preferably not lower than 95%, and particularly preferably not lower than 97%.

A particularly preferable dry-coated tablet of the present invention is: a dry-coated tablet having an outer shell that causes the dry-coated tablet to have a friability (accumulative number of rotations: 100 rotations) of not higher than 0.3%, wherein the disintegration time of the dry-coated tablet determined by the Disintegration Test described in the General Tests, Processes and Apparatus section of the Japanese Pharmacopoeia is not more than 120 seconds, and is further preferably a dry-coated tablet having an outer shell that causes the dry-coated tablet to have a friability (accumulative number of rotations: 100 rotations) of not higher than 0.2%, wherein the disintegration time of the dry-coated tablet determined by the Disintegration Test described in the General Tests, Processes and Apparatus section of the Japanese Pharmacopoeia is not more than 95 seconds;
further preferably, a dry-coated tablet having an outer shell that causes the dry-coated tablet to have a friability (accumulative number of rotations: 100 rotations) of not higher than 0.2% and that substantially does not become cracked or chipped after a drop test from a height of 1 m, wherein the disintegration time of the dry-coated tablet determined by the Disintegration Test described in the General Tests, Processes and Apparatus section of the Japanese Pharmacopoeia is not more than 95 seconds, and is further preferably a dry-coated tablet having an outer shell that causes the dry-coated tablet to have a friability (accumulative number of rotations: 100 rotations) of not higher than 0.2% and that substantially does not become cracked or chipped after a drop test from a height of 2 m, wherein the disintegration time of the dry-coated tablet determined by the Disintegration Test described in the General Tests, Processes and Apparatus section of the Japanese Pharmacopoeia is not more than 95 seconds; and
further preferably, a dry-coated tablet having an outer shell that causes the dry-coated tablet to have a friability (accumulative number of rotations: 100 rotations) of not higher than 0.2% and that substantially does not become cracked or chipped after a drop test from a height of 2 m, wherein the disintegration time of the dry-coated tablet determined by the Disintegration Test described in the General Tests, Processes and Apparatus section of Japanese Pharmacopoeia is not more than 95 seconds, wherein the disintegration time of the dry-coated tablet measured using the orally disintegrating tablet tester ODT-101 is not more than 20 seconds, and wherein the dissolution rate (CDHP, 15-minute value) measured by Method 2 (50 min⁻¹, test liquid: 900 mL of water) of the Dissolution Test described in the General Tests, Processes and Apparatus section in the Japanese Pharmacopoeia is not lower than 90%, and is particularly preferably a dry-coated tablet having an outer shell that causes the dry-coated tablet to have a friability (accumulative number of rotations: 100 rotations) of not higher than 0.2% and that substantially does not become cracked or chipped after a drop test from a height of 2 m, wherein a disintegration time of the dry-coated tablet determined by the Disintegration Test described in the General Tests, Processes and Apparatus section of the Japanese Pharmacopoeia is not more than 95 seconds, wherein the disintegration time of the dry-coated tablet measured using the orally disintegrating tablet tester ODT-101 is not more than 16 seconds, and wherein a dissolution rate (CDHP, 15-minute value) measured by Method 2 (50 min⁻¹, test liquid: 900 mL of water) of the Dissolution Test described in the General Tests, Processes and Apparatus section of the Japanese Pharmacopoeia is not lower than 95%.

Furthermore, "hardness" is an index for evaluating tablet strength, and can be measured in accordance with the Tablet Breaking Force method described in the USP. That is, the load applied when a tablet breaks is measured by applying pressure thereto at a constant rate (not higher than 20 N/second or not higher than 3.5 mm/second) between two parallel pressure plates, in a diameter direction when the tablet is a round tablet. The hardness of the dry-coated tablet of the present invention is, in view of balance with disintegration, preferably 30 N to 60 N, and more preferably 35 N to 55 N.

The additive included in the outer shell of the dry-coated tablet of the present invention for improving tablet strength is crystalline cellulose. Examples of further additives included in the outer shell for improving tablet strength include lactose, hydroxypropyl cellulose, and the like. However, the additive is preferably a mixture of crystalline cellulose and lactose. Furthermore, a disintegrant for enhancing disintegration ability and dissolution characteristics is preferably included, and examples thereof include crospovidone, partially pregelatinized starch, carmellose, corn starch, low-substituted hydroxypropyl cellulose, and the like. More preferably, crospovidone and/or partially pregelatinized starch is included as a disintegrant.

In the present invention, the amount of lactose used in outer shell components is preferably 30 to 65 mass%, more preferably 40 to 60 mass%, and particularly preferably 45 to 55 mass%. If the amount of lactose is smaller than 30 mass%, the disintegration ability deteriorates; and if it is larger than 65 mass%, friability tends to become large. The amount of crystalline cellulose used is preferably 30 to 50 mass%, and more preferably 35 to 45 mass%. If the amount of crystalline cellulose is smaller than 30 mass%, friability becomes large; and if it is larger than 50 mass%, disintegration ability deteriorates and mouthfeel tends to worsen. In addition, with regard to the quantitative ratio of lactose and crystalline cellulose, when a balance among hardness, disintegration ability, and ingestion sensation of the tablet is taken into consideration, mass ratio of the amount of lactose and the amount of crystalline cellulose is more preferably set at 1:0.5 to 1.5, and particularly preferably set at 1:0.8 to 1.3. The amount of crospovidone used is preferably 2.5 to 15 mass%, and more preferably 2.5 to 7.5 mass%. The amount of partially pregelatinized starch used is preferably 2 to 7.5 mass%, and more preferably 2.5 to 5 mass%.

The inner core of the dry-coated tablet of the present invention can be formed only from active ingredients consisting of tegafur, gimeracil, and oteracil potassium. The blended amount of the active ingredients may account for 50 mass% to 100 mass% of the components of the inner core, preferably 70 mass% to 99 mass%. Furthermore, in order to provide adequate tablet strength and disintegration ability, pharmaceutically acceptable binders and/or disintegrants may be added in an adequate amount. The binders are not particularly limited as long as they are binders used for medical preparations, and examples thereof include starch paste liquid, methyl cellulose, hydroxypropyl cellulose (HPC-SSL, HPC-SL, HPC-L, etc.), hydroxypropyl methylcellulose, sodium carboxymethyl cellulose (carmellose sodium), gum arabic, gelatin, agar, tragacanth, sodium alginate, pullulan, polyvinylpyrrolidone, polyvinyl alcohol, polyethylene glycol, and the like. These binders may be used singly or in a combination of two or more. Among those, hydroxypropyl cellulose is preferable. The contained amount of the binder with respect to the total amount of tegafur, gimeracil, and oteracil potassium may be 0.1 to 2.0 mass%, and more preferably 0.4 to 1.0 mass%. Furthermore, examples of the disintegrant include carmellose, carmellose calcium, croscarmellose sodium, crystalline cellulose, low-substituted hydroxypropyl cellulose, crospovidone, and the like. These disintegrants may be used singly or in a combination of two or more. Among those, crospovidone is preferable. The contained amount of the disintegrant with respect to the total amount of tegafur, gimeracil, and oteracil potassium is preferably 3 to 15 mass%.

In addition, a small amount of a disintegration aid may be added to the inner core, and examples of the disintegration aid include carmellose, carmellose calcium, carmellose sodium, croscarmellose sodium, low-substituted hydroxypropyl cellulose, sodium carboxymethyl starch, corn starch, potato starch, pregelatinized starch, partially pregelatinized starch, hydroxypropyl starch, crospovidone, sodium lauryl sulfate, polysorbate, polyoxyethylene polyoxypropylene glycol, sorbitan monooleate, propylene glycol monostearate, polyethylene glycol monolaurate, and the like. These disintegration aids may be used singly or in a combination of two or more.

Other than the additives described above, the dry-coated tablet of the present invention may include various additives that are generally used for pharmaceutical preparations, as long as the advantageous effect of the present invention is not adversely affected. The additives for pharmaceutical preparations are not particularly limited as long as they are additives that are generally used, and examples thereof include lubricants, coloring agents, flavours, corrigents, and the like. Examples of the lubricants include magnesium stearate, calcium stearate, stearic acid, sodium stearyl fumarate, talc, sucrose fatty acid ester, and the like. Examples of the coloring agents include food dye yellow No. 5, food dye red No. 2, food dye blue No. 2, food lake dye, yellow ferric oxide, titanium oxide, and the like. Examples of the flavours include various fragrances of orange, lemon, etc. Examples of the corrigents include L-menthol, camphor, peppermint, and the like.

The dry-coated tablet of the present invention can be manufactured using, for example, dry-coat tableting machines such as a LIBRA2 DC tableting machine (manufactured by Kikusui Seisakusho, Ltd.) and an AP-MS-C-type dry-coat tableting machine (manufactured by Hata Iron Works Co., Ltd.); and further using an Autograph AG-E50k (manufactured by Shimadzu Corp.), etc. More specifically, a general manufacturing method consists largely of four stages: (i) supplying one portion of outer shell components in a die; (ii) supplying an inner core pre-formed as a tablet in the die; (iii) supplying the remaining outer shell components to the die; and (iv) compacting those in the die between upper and lower punches to manufacture a dry-coated tablet. Furthermore, the manufacturing may be conducted using methods disclosed in WO200328706, J. Jpn. Soc. Pharm. Mach. & Eng. 14(4), 12-21 (2005), etc., and a rotary compression molding machine (manufactured by Sanwa Kagaku Kenkyusho Co., Ltd.). The manufacturing method is preferable since a tablet having a double structure of the inner core and the outer shell can be constructed easily with high productivity. More specifically, the dry-coated tablet of the present invention can be easily manufactured with compression means that include punches both above and below a die, wherein at least an upper punch has a double structure of a center punch and an outer punch; and the center punch and the outer punch are both slidable, and can perform a compression operation. In this manner, since the dry-coated tablet of the present invention can be manufactured at one time with a single tableting machine, there is no need to implement complicated techniques or steps as in hitherto known art, and the dry-coated tablet can be manufactured efficiently. Therefore, deviation of positions of a core as observed in hitherto known dry-coated tablets does not occur, and it becomes possible to form an extremely thin outer shell. In particular, the thickness of the outer shell can be made to be not larger than 1 mm. Making the outer shell thin also contributes to improving the disintegration ability of the dry-coated tablet.

The shape of the dry-coated tablet of the present invention is not particularly limited as long as the shape is easy to hold, and does not cause any discomfort when ingested. Similar to general pharmaceutical products, a pharmaceutical preparation having a round or oval shape is preferable. Furthermore, the pharmaceutical preparation may have a size that can be placed inside in the mouth and that does not accompany difficulty in chewing. For example, in the case of a round tablet, a size of not larger than 25 mm in diameter is sufficient, and the round tablet may be designed to be 4 to 25 mm in diameter, preferably 6 to 16 mm in diameter, further preferably 7 to 12 mm in diameter. A height of not larger than 10 mm is sufficient for the preparation, and the height may be designed to be 1 to 10 mm, preferably 1.5 to 7 mm, and further preferably 2 to 5 mm.

The shape of the inner core depends on the punch-tip shape of the punch that is used, and conforms to the shape of the dry-coated tablet. The size of the inner core often depends on the size of the entire dry-coated tablet, and an overly small size is not preferable for smoothly performing a step for molding the inner core. In addition, in order to improve disintegration ability of the dry-coated tablet, the inner core is preferably formed to be large with respect to the outer shell, as long as molding of the outer shell is not hindered. Therefore, in the case of a round tablet, a diameter of not larger than 24 mm is sufficient for the inner core, and the inner core may be designed to be 3 to 24 mm in diameter, preferably 5 to 15 mm in diameter, and further preferably 6 to 11 mm in diameter. If necessary, the inner core can be divided into multiple parts.

The thickness of the outer shell may be set at a thickness that conforms to the size of the inner core, results in low friability, and allows the shape of the dry-coated tablet to be maintained by the outer shell; and a thickness in the range of 0.2 to 2 mm is suitable. In order to enhance rapid disintegration in the mouth, it is better not to increase the tablet strength of the outer shell components more than necessary. Therefore, as long as problems related to friability do not occur, i.e., the shape of the dry-coated tablet can be maintained, the thickness of the outer shell is preferably set as thin as possible. Setting the thickness to 0.5 to 1.5 mm is realistic and preferable.

As the dry-coated tablet of the present invention, a dry-coated tablet whose inner core is 3 to 24 mm in diameter, whose outer shell has a thickness of 0.2 to 2 mm, and whose preparation has a height of 1 to 10 mm is preferable; a dry-coated tablet whose inner core is 5 to 15 mm in diameter, whose outer shell has a thickness of 0.5 to 1.5 mm, and whose preparation has a height of 1.5 to 7 mm is more preferable; and a dry-coated tablet whose inner core is 6 to 11 mm in diameter, whose outer shell has a thickness of 0.5 to 1.5 mm, and whose preparation has a height of 2 to 5 mm is particularly preferable.

### Examples

The present invention is described in further detail with reference to Reference Examples, Examples, and Experimental Examples; however, the present invention is not limited thereto. It should be noted that the tegafur and gimeracil used in the Examples are manufactured by Taiho Pharmaceutical Co., Ltd.; and that the oteracil potassium used in the Examples is manufactured by Sumitomo Chemical Co., Ltd. Furthermore, the hydroxypropyl cellulose (HPC) is manufactured by Nippon Soda Co., Ltd., the partially pregelatinized starch and crystalline cellulose are manufactured by Asahi Kasei Chemicals Corp., the magnesium stearate is manufactured by Taihei Chemical Industrial Co., Ltd., and the crospovidone is manufactured by BASF.

### Reference Example 1 (Preparation of inner core components 1)

Using a fluidized bed granulator multiplex MP-01 (manufactured by Powrex Corp.), 200.0 g of water was sprayed on a mixture of 66.1 g of tegafur, 19.15 g of gimeracil, 64.75 g of oteracil potassium, and 150 g of lactose (manufactured by Borculo Domo Ingredients Ltd.) for granulation to prepare inner core components.

### Reference Example 2 (Preparation of inner core components 2)

In the same manner as in Reference Example 1, a solution obtained by dissolving 1.0 g of HPC in 199.0 g of water was sprayed on a mixture of 66.1 g of tegafur, 19.15 g of gimeracil, 64.75 g of oteracil potassium, and 150 g of lactose (manufactured by Borculo Domo Ingredients Ltd.) for granulation to prepare inner core components.

### Reference Example 3 (Preparation of inner core components 3)

In the same manner as in Reference Example 1, 200 g of water was sprayed on a mixture of 132.2 g of tegafur, 38.3 g of gimeracil, and 129.5 g of oteracil potassium for granulation to prepare inner core components.

### Reference Example 4 (Preparation of inner core components 4)

In the same manner as in Reference Example 1, a solution obtained by dissolving 0.3 g of HPC in 199.7 g of water was sprayed on a mixture of 132.2 g of tegafur, 38.3 g of gimeracil, and 129.5 g of oteracil potassium for granulation to prepare inner core components.

### Reference Example 5 (Preparation of inner core components 5)

In the same manner as in Reference Example 1, a solution obtained by dissolving 1.5 g of HPC in 198.5 g of water was sprayed on a mixture of 132.2 g of tegafur, 38.3 g of gimeracil, and 129.5 g of oteracil potassium for granulation to prepare inner core components.

### Reference Example 6 (Preparation of inner core components 6)

In the same manner as in Reference Example 1, a solution obtained by dissolving 3.0 g of HPC in 197.0 g of water was sprayed on a mixture of 132.2 g of tegafur, 38.3 g of gimeracil, and 129.5 g of oteracil potassium for granulation to prepare inner core components.

### Reference Example 7 (Preparation of inner core components 7)

In the same manner as in Reference Example 1, a solution obtained by dissolving 4.5 g of HPC in 195.5 g of water was sprayed on a mixture of 132.2 g of tegafur, 38.3 g of gimeracil, and 129.5 g of oteracil potassium for granulation to prepare inner core components.

### Reference Example 8 (Preparation of inner core components 8)

In the same manner as in Reference Example 1, a solution obtained by dissolving 6.0 g of HPC in 194.0 g of water was sprayed on a mixture of 132.2 g of tegafur, 38.3 g of gimeracil, and 129.5 g of oteracil potassium for granulation to prepare inner core components.

### Reference Example 9 (Preparation of inner core components 9)

In the same manner as in Reference Example 1, a solution obtained by dissolving 4 g of HPC in 396 g of water was sprayed on a mixture of 200 g of tegafur, 58 g of gimeracil, 196 g of oteracil potassium, and 14 g of partially pregelatinized starch (manufactured by Asahi Kasei Chemicals Corp.) for granulation to prepare inner core components.

### Reference Example 10 (Preparation of inner core components 10)

In the same manner as in Reference Example 1, a solution obtained by dissolving 4 g of HPC in 396 g of water was sprayed on a mixture of 200 g of tegafur, 58 g of gimeracil, and 196 g of oteracil potassium for granulation to prepare inner core components.

### Example 1

Using an Autograph AG-E50k (manufactured by Shimadzu Corp.), an outer shell that was a mixture of 59.88 mg of lactose (manufactured by Meggle Excipients & Technology), 60 mg of crystalline cellulose, and 0.12 mg of magnesium stearate, and an inner core that was a mixture of 57 mg of the granulated substance of Reference Example 5 (tegafur: 25 mg), 2.84 mg of partially pregelatinized starch, and 0.28 mg of magnesium stearate, a dry-coated tablet having a tablet outer diameter of 8 mm was prepared by sequentially packing 30 mg of a first layer of the outer shell, 60.12 mg of the inner core, and 60 mg of a second layer of the outer shell, and applying a tableting pressure of 5 kN. Fig. 1 shows a schematic diagram of a cross section of the obtained dry-coated tablet. The diameter of the inner core of the obtained dry-coated tablet was 6 mm, and a thickness (a) of the outer shell was 1 mm.

### Example 2

In the same manner as Example 1, using an Autograph AGE50k (manufactured by Shimadzu Corp.), an outer shell that was a mixture at a proportion of 53.76 mg of lactose (manufactured by MEGGLE Excipients & Technology), 60 mg of crystalline cellulose, 6 mg of crospovidone, and 0.24 mg of magnesium stearate, and an inner core that was a mixture of 57 mg of the granulated substance of Reference Example 5 (tegafur: 25 mg), 2.84 mg of partially pregelatinized starch, and 0.28 mg of magnesium stearate, a dry-coated tablet containing 25 mg of tegafur, 120 mg of the outer shell, and 60.12 mg of the inner core was prepared by applying a tableting pressure of 5 kN. The outer diameter of the obtained dry-coated tablet was 8 mm, the diameter of the inner core was 6 mm, and the thickness of the outer shell was 1 mm.

### Example 3

Using an Autograph AG-E50k (manufactured by Shimadzu Corp.), the target dry-coated tablets were prepared in the same manner as in Example 1, except that the inner core components of Reference Examples 1 to 4 and 6 to 10 were used.

### Example 4

Using a rotary compression molding machine (manufactured by Sanwa Kagaku Kenkyusho Co., Ltd.) as described in WO200328706, an outer shell that was a mixture at a proportion shown in Table 1, and inner core components that were a mixture of 57.25 mg of the granulated substance of Reference Example 10 (amount of tegafur: 25 mg), 1.5 mg of crospovidone, 1.5 mg of partially pregelatinized starch, 1.5 mg of corrigent, and 0.25 mg of magnesium stearate, dry-coated tablets 1 to 4 having a tablet outer diameter of 8 mm were prepared by sequentially packing the first layer of the outer shell, 62 mg of the inner core, and the second layer of the outer shell (amount of the first layer of the outer shell:amount of the second layer of the outer shell = 35:85), and applying a tableting pressure of 4 kN.

### Experimental Example 1

Hardness, friability, and oral disintegration and dissolution tests were conducted for the dry-coated tablets 1 to 4 obtained in Example 4. The results are shown in Table 1. The results confirmed that a dry-coated tablet having excellent hardness, friability, oral disintegration characteristics, and dissolution characteristics can be obtained when the outer shell components includes 34 to 64 mass% of lactose for direct tableting, 30 to 50 mass% of crystalline cellulose, and 5 to 15 mass% of crospovidone.

### <Test conditions>

Hardness: Tablet Tester 8M tablet hardness tester, manufactured by Schleuniger.
Friability: Tablet friability tester; 100 rotations (25 rpm, 4 minutes).
Disintegration test: Disintegration Test described in the General Tests, Processes and Apparatus section of the Japanese Pharmacopoeia; the test liquid was water.
Oral disintegration test: Oral disintegration tester ODT-101, manufactured by Toyama Chemical Co., Ltd.; 20 g weight having a diameter of 20 mm; number of rotations was 75 rpm; test liquid was water.
Dissolution test: Method 2 (50 min⁻¹) of the Dissolution Test described in the General Tests, Processes and Apparatus section of the Japanese Pharmacopoeia; the test liquid was 900 mL of water.

**[Table 1]**

| Outer shell components | Dry-coated tablet 1 | Dry-coated tablet 2 | Dry-coated tablet 3 | Dry-coated tablet 4 |
|---|---|---|---|---|
| Lactose (manufactured by DMV-Fonterra Excipients) (mg) | 76.75 | 52.75 | 58.75 | 40.75 |
| Crystalline cellulose (mg) | 36 | 60 | 48 | 60 |
| Crospovidone (mg) | 6 | 6 | 12 | 18 |
| Magnesium stearate (mg) | 0.25 | 0.25 | 0.25 | 0.25 |
| Corrigent, Flavour, Color | q.s. | q.s. | q.s. | q.s. |
| Total (mg) | 120 | 120 | 120 | 120 |
| Hardness (N) n=3 | 41, 41, 41 | 52, 56, 57 | 51, 52, 54 | 53, 57, 58 |
| Friability (%); 100 rotations | 0.04 | 0.01 | 0.00 | 0.00 |
| Disintegration test (s) | 77.8 | 68.5 | 78.1 | 71.6 |
| Oral disintegration tester (s) | 13.3 | 13.1 | 15.1 | 14.5 |
| Dissolution (%); CDHP, 15-minute value | 97.0 | 95.8 | 96.9 | 94.5 |

### Example 5

Using the same rotary compression molding machine (manufactured by Sanwa Kagaku Kenkyusho Co., Ltd.) as that used in Example 4, an outer shell that was a mixture at a proportion shown in Table 2, and inner core components prepared in the same manner as that in Example 4, dry-coated tablets 5 to 8 having a tablet outer diameter of 8 mm were prepared by sequentially packing the first layer of the outer shell, 60 mg of the inner core, and the second layer of the outer shell (amount of the first layer of the outer shell:amount of the second layer of the outer shell = 35:85), and applying a tableting pressure of 4 kN.

### Experimental Example 2

Hardness, friability, and oral disintegration and dissolution tests were conducted for the dry-coated tablets 5 to 8 obtained in Example 5. The results are shown in Table 2. The results confirmed that a dry-coated tablet having excellent hardness, friability, oral disintegration characteristics, and dissolution characteristics can be obtained when 39 to 59 mass% of lactose for direct tableting, 30 to 50 mass% of crystalline cellulose, 2.5 to 7.5 mass% of crospovidone, and 2.5 to 7.5 mass% of partially pregelatinized starch are included.

### <Test conditions>

Hardness: Tablet Tester 8M tablet hardness tester, manufactured by Schleuniger.
Friability: PTF30ERA tablet friability tester manufactured by Pharma Test Apparatebau; 100 rotations (25 rpm, 4 minutes). Disintegration test: Disintegration Test described in the General Tests, Processes and Apparatus section of the Japanese Pharmacopoeia; the test liquid was water.
Oral disintegration test: ODT-101 oral disintegration tester manufactured by Toyama Chemical Co., Ltd.; 20 g weight, diameter of 20 mm; number of rotations was 75 rpm; the test liquid was water.
Dissolution test: Method 2 (50 min⁻¹) of the Dissolution Test described in the General Tests, Processes and Apparatus section of the Japanese Pharmacopoeia; the test liquid was 900 mL of water.

**[Table 2]**

| Outer shell components | Dry-coated tablet 5 | Dry-coated tablet 6 | Dry-coated tablet 7 | Dry-coated tablet 8 |
|---|---|---|---|---|
| Lactose (manufactured by DMV-Fonterra Excipients) (mg) | 58.75 | 70.75 | 46.75 | 46.75 |
| Crystalline cellulose (mg) | 48 | 36 | 60 | 60 |
| Crospovidone (mg) | 6 | 9 | 9 | 3 |
| Partially pregelatinized starch (mg) | 6 | 3 | 3 | 9 |
| Magnesium stearate (mg) | 0.25 | 0.25 | 0.25 | 0.25 |
| Corrigent, Flavour, Color | q.s. | q.s. | q.s. | q.s. |
| Total (mg) | 120 | 120 | 120 | 120 |
| Hardness (N); n=3 | 40, 40, 43 | 41, 42, 44 | 41, 43, 44 | 35, 35, 37 |
| Friability (%); 100 rotations | 0.0 | 0.12 | 0.06 | 0.13 |
| Disintegration test (s) | 70.6 | 91.8 | 73.6 | 66.9 |
| Oral disintegration tester (s) | 13.3 | 15.4 | 11.3 | 14.8 |
| Dissolution (%); CDHP, 15-minute value | 97.0 | 97.3 | 92.3 | 99.9 |

### Example 6

Using the same rotary compression molding machine (manufactured by Sanwa Kagaku Kenkyusho Co., Ltd.) as that used in Example 4, an outer shell that was a mixture at a proportion shown in Table 3, and inner core components that were a mixture of 45.8 mg of the granulated substance of Reference Example 10 (amount of tegafur: 20 mg), 2 mg of crospovidone, 0.4 mg of partially pregelatinized starch, 1.2 mg of corrigent, and 0.2 mg of magnesium stearate, dry-coated tablets 9 to 12 having a tablet outer diameter of 8 mm were prepared by sequentially packing the first layer of the outer shell, 49.6 mg of the inner core, and the second layer of the outer shell (amount of the first layer of the outer shell:amount of the second layer of the outer shell = 30:66), and applying a tableting pressure of 4 kN.

### Experimental Example 3

Hardness, friability, and oral disintegration and dissolution tests were conducted for the dry-coated tablets 9 to 12 obtained in Example 6. The results are shown in Table 3. The results confirmed that a dry-coated tablet having excellent hardness, friability, oral disintegration characteristics, and dissolution characteristics can be obtained when 39 to 59 mass% of lactose for direct tableting, 30 to 50 mass% of crystalline cellulose, 2.5 to 7.5 mass% of crospovidone, and 2.5 to 7.5 mass% of partially pregelatinized starch are included.

**[Table 3]**

| Outer shell components | Dry-coated tablet 9 | Dry-coated tablet 10 | Dry-coated tablet 11 | Dry-coated tablet 12 |
|---|---|---|---|---|
| Lactose (manufactured by DMV-Fonterra Excipients) (mg) | 47 | 56.6 | 37.4 | 37.4 |
| Crystalline cellulose (mg) | 38.4 | 28.8 | 48 | 48 |
| Crospovidone (mg) | 4.8 | 7.2 | 7.2 | 2.4 |
| Partially pregelatinized starch (mg) | 4.8 | 2.4 | 2.4 | 7.2 |
| Magnesium stearate (mg) | 0.2 | 0.2 | 0.2 | 0.2 |
| Corrigent, Flavour, Color | q.s. | q.s. | q.s. | q.s. |
| Total (mg) | 96 | 96 | 96 | 96 |

### Experimental Example 4

A tablet drop test in which a tablet is made to undergo a gravity-drop from a height of 1 m onto a stainless steel plate was conducted on the dry-coated tablets 6 to 8 obtained in Example 5, and on commercially available orally disintegrating tablets (company A, company B, company C, company D, and company E). The results are shown in Table 4 and Table 5. The results confirmed that the dry-coated tablets 5 to 8 were excellent dry-coated tablets in terms of cracks and chips; as well as mass reduction rate, when compared to the commercially available orally disintegrating tablets.

### <Test conditions>

Tablet drop test: Stainless steel plate; a height of 1 m (five pieces).

**[Table 4]**

| | | Dry-coated tablet 6 | Dry-coated tablet 7 | Dry-coated tablet 8 |
|---|---|---|---|---|
| Drop distance: 1 m | Number of chipped tablets in 5 tablets | 0 | 0 | 0 |
| | Mass reduction rate (%) | 0.054 | 0.055 | 0.065 |
| Drop distance: 2 m | Number of chipped tablets in 5 tablets | 0 | 0 | 0 |
| | Mass reduction rate (%) | 0.054 | 0.077 | 0.076 |

**[Table 5]**

| Comparative Example | | | | | | |
|---|---|---|---|---|---|---|
| | | A | B | C | D | E |
| Drop distance: 1 m | Number of chipped tablets in 5 tablets | 0 | 0 | 1 | 0 | 3 |
| | Mass reduction rate (%) | 0.12 | 0.1 | 10.13 | 0.1 | 0.13 |
| Drop distance: 2 m | Number of chipped tablets in 5 tablets | 0 | 1 | 4 | 0 | 4 |
| | Mass reduction rate (%) | 0.12 | 0.13 | 33.77 | 0.12 | 0.48 |

## Claims

1. A dry-coated tablet comprising:
an inner core containing, as active ingredients, (a) tegafur, (b) gimeracil, and (c) oteracil potassium, and
an outer shell containing crystalline cellulose,
wherein the dry-coated tablet is an orally disintegrating tablet.

2. The dry-coated tablet according to claim 1, containing (a) tegafur, (b) gimeracil, and (c) oteracil potassium at a molar ratio of 1:0.4:1.

3. The dry-coated tablet according to claim 1 or 2, wherein the active ingredients consisting of (a) tegafur, (b) gimeracil, and (c) oteracil potassium account for 50 mass% to 100 mass% of components of the inner core, preferably 70 mass% to 99 mass%.

4. The dry-coated tablet according to any one of claims 1 to 3, wherein the total content of the active ingredients (a) to (c) accounts for 10 to 60 mass% of the dry-coated tablet.

5. The dry-coated tablet according to any one of claims 1 to 4, wherein the outer shell further contains one or two compounds among lactose and hydroxypropyl cellulose.

6. The dry-coated tablet according to claim 5, wherein the outer shell further contains lactose.

7. The dry-coated tablet according to claim 5 or 6, wherein the outer shell further contains a disintegrant.

8. The dry-coated tablet according to claim 7, wherein the disintegrant is selected from the group consisting of crospovidone, carmellose, corn starch, low-substituted hydroxypropyl cellulose, and partially pregelatinized starch, preferably, crospovidone and/or partially pregelatinized starch.

9. The dry-coated tablet according to any one of claims 1 to 8, wherein the outer shell contains 30 to 65 mass% lactose.

10. The dry-coated tablet according to any one of claims 1 to 9, wherein the outer shell contains 30 to 50 mass% crystalline cellulose.

11. The dry-coated tablet according to any one of claims 1 to 10, wherein the outer shell contains 2.5 to 15 mass% crospovidone.

12. The dry-coated tablet according to any one of claims 1 to 11, wherein the outer shell contains 2 to 7.5 mass% partially pregelatinized starch.

## Patentansprüche

1. Trockenbeschichtete Tablette, umfassend:
einen inneren Kern, der als Wirkstoffe (a) Tegafur, (b) Gimeracil und (c) Oteracil-Kalium enthält, und
eine äußere Hülle, die kristalline Cellulose enthält,
wobei die trockenbeschichtete Tablette eine oral auflösbare Tablette ist.

2. Trockenbeschichtete Tablette gemäß Anspruch 1, enthaltend (a) Tegafur, (b) Gimeracil und (c) Oteracil-Kalium in einem Molverhältnis von 1:0,4:1.

3. Trockenbeschichtete Tablette gemäß Anspruch 1 oder 2, wobei die Wirkstoffe, bestehend aus (a) Tegafur, (b) Gimeracil und (c) Oteracil-Kalium, 50 Massen-% bis 100 Massen-% der Komponenten des inneren Kerns ausmachen, bevorzugt 70 Massen-% bis 99 Massen-%.

4. Trockenbeschichtete Tablette gemäß einem der Ansprüche 1 bis 3, wobei der Gesamtgehalt der Wirkstoffe (a) bis (c) 10 bis 60 Massen-% der trockenbeschichteten Tablette ausmacht.

5. Trockenbeschichtete Tablette gemäß einem der Ansprüche 1 bis 4, wobei die äußere Hülle zusätzlich eine oder zwei Verbindungen von Lactose und Hydroxypropylcellulose enthält.

6. Trockenbeschichtete Tablette gemäß Anspruch 5, wobei die äußere Hülle zusätzlich Lactose enthält.

7. Trockenbeschichtete Tablette gemäß Anspruch 5 oder 6, wobei die äußere Hülle zusätzlich ein Sprengmittel enthält.

8. Trockenbeschichtete Tablette gemäß Anspruch 7, wobei das Sprengmittel aus der Gruppe ausgewählt ist, die aus Crospovidon, Carmellose, Maisstärke, niedrig substituierter Hydroxypropylcellulose und teilweise vorgelatinierter Stärke, bevorzugt Crospovidon und/oder teilweise vorgelatinierter Stärke, besteht.

9. Trockenbeschichtete Tablette gemäß einem der Ansprüche 1 bis 8, wobei die äußere Hülle 30 bis 65 Massen-% Lactose enthält.

10. Trockenbeschichtete Tablette gemäß einem der Ansprüche 1 bis 9, wobei die äußere Hülle 30 bis 50 Massen-% kristalline Cellulose enthält.

11. Trockenbeschichtete Tablette gemäß einem der Ansprüche 1 bis 10, wobei die äußere Hülle 2,5 bis 15 Massen-% Crospovidon enthält.

12. Trockenbeschichtete Tablette gemäß einem der Ansprüche 1 bis 11, wobei die äußere Hülle 2 bis 7,5 Massen-% teilweise vorgelatinierte Stärke enthält.

## Revendications

1. Comprimé enrobé à sec comprenant :
un noyau interne contenant, en tant qu'ingrédients actifs, (a) du tégafur, (b) du giméracil et (c) de l'oteracil potassium, et
une enveloppe externe contenant de la cellulose cristalline,
dans lequel le comprimé enrobé à sec est un comprimé orodispersible.

2. Comprimé enrobé à sec selon la revendication 1, contenant (a) du tégafur, (b) du giméracil, et (c) de l'oteracil potassium dans un rapport molaire de 1:0, 4:1.

3. Comprimé enrobé à sec selon la revendication 1 ou 2, dans lequel les ingrédients actifs consistant en (a) tégafur, (b) giméracil et (c) oteracil potassium représentent 50 % en masse à 100 % en masse des composants du noyau interne, de préférence 70 % en masse à 99 % en masse.

4. Comprimé enrobé à sec selon l'une quelconque des revendications 1 à 3, dans lequel la teneur totale en ingrédients actifs (a) à (c) représente de 10 à 60 % en masse du comprimé enrobé à sec.

5. Comprimé enrobé à sec selon l'une quelconque des revendications 1 à 4, dans lequel l'enveloppe externe contient un ou deux composés parmi le lactose et l'hydroxypropyl cellulose.

6. Comprimé enrobé à sec selon la revendication 5, dans lequel l'enveloppe externe contient en outre du lactose.

7. Comprimé enrobé à sec selon la revendication 5 ou 6, dans lequel l'enveloppe externe contient en outre un désintégrant.

8. Comprimé enrobé à sec selon la revendication 7, dans lequel le désintégrant est choisi dans le groupe constitué par la crospovidone, la carmellose, l'amidon de maïs, l'hydroxypropyl cellulose faiblement substituée, et l'amidon partiellement prégélatinisé, de préférence, la crospovidone et/ou l'amidon partiellement prégélatinisé.

9. Comprimé enrobé à sec selon l'une quelconque des revendications 1 à 8, dans lequel l'enveloppe externe contient de 30 à 65 % en masse de lactose.

10. Comprimé enrobé à sec selon l'une quelconque des revendications 1 à 9, dans lequel l'enveloppe externe contient de 30 à 50 % en masse de cellulose cristalline.

11. Comprimé enrobé à sec selon l'une quelconque des revendications 1 à 10, dans lequel l'enveloppe externe contient de 2,5 à 15 % en masse de crospovidone.

12. Comprimé enrobé à sec selon l'une quelconque des revendications 1 à 11, dans lequel l'enveloppe externe contient de 2 à 7,5 % en masse d'amidon partiellement prégélatinisé.
